# EUROPÄISCHE PATENTSCHRIFT

(11) **EP 1 457 175 B1**
(45) Veröffentlichungstag und Bekanntmachung des Hinweises auf die Patenterteilung: **07.03.2007**
(21) Anmeldenummer: 04004290.5
(22) Anmeldetag: 26.02.2004
(51) Int. Cl.: A61F 2/68

(54) **Orthopädietechnisches Hilfsmittel mit einer Verriegelungsvorrichtung**
Orthopaedic device with locking mechanism.
Dispositif orthopédique avec méchanisme de verrouillage.

(30) Priorität: 12.03.2003 DE 10311187
(43) Veröffentlichungstag der Anmeldung: 15.09.2004
(73) Patentinhaber: Otto Bock HealthCare GmbH, 37115 Duderstadt (DE)
(72) Erfinder: Lidolt, Klaus, 37115 Duderstadt (DE); Schilling, Matthias, 37345 Weissenborn-Lüderode (DE)
(74) Vertreter: Lins, Edgar

(56) Entgegenhaltungen:
- EP-A- 0 141 640
- EP-A- 0 380 060
- DE-A- 2 314 510
- GB-A- 1 386 942
- GB-A- 2 280 609
- DIETL H ET AL: "DER EINSATZ VON ELEKTRONIK BEI PROTHESEN ZUR VERSORGUNG DER UNTERENEXTREMITAET" MEDIZINISCH ORTHOPADISCHE TECHNIK, GENTNER VERLAG. STUTTGART, DE, Bd. 117, Nr. 1, 1997, Seiten 31-35, XP000679254 ISSN: 0340-5508

## Beschreibung

Die Erfindung betrifft ein orthopädietechnisches Hilfsmittel mit zwei relativ zueinander bewegbaren Teilen und einer Verriegelungsvorrichtung zum Verriegeln der beiden Teile in einer vorbestimmten relativen Position und zum Entriegeln der Teile zur Freigabe der Bewegung der Teile zueinander, wobei die Verriegelungsvorrichtung von einem Steuermodul aus elektromechanisch betätigbar ist.

Orthopädietechnische Hilfsmittel dieser Art werden für zahlreiche Anwendungen eingesetzt, um am menschlichen Körper vorübergehend oder dauernd vorhandene Schwächen zu kompensieren und sonst nicht ausübbare Funktionen zu ermöglichen. Dies geschieht dadurch, dass das orthopädietechnische Hilfsmittel eine Stützfunktion ausübt, indem in einer bestimmten Stellung der Teile des Hilfsmittels diese Teile zueinander verriegelt werden, wobei die verriegelte Stellung der beiden Teile zueinander einer Gebrauchsstellung entspricht, in der der betreffende Patient die Stützfunktion durch das orthopädietechnische Hilfsmittel benötigt. Ein bevorzugter Anwendungsfall eines derartigen orthopädietechnischen Hilfsmittels ist die Ausbildung als Gelenkorthese, wobei beispielsweise die durch ein Gelenk miteinander verbundenen Teile der Gelenkorthese in gestreckter Stellung verriegelbar sind, um beispielsweise die Funktion einer Extremität in der gestreckten und verriegelten Stellung der Gelenkorthese zu ermöglichen. Um in eine Ruhestellung zu gelangen, muss die Verriegelungsvorrichtung entriegelt werden. Dies geschieht beispielsweise bei bekannten Kniegelenkorthesen durch einen Bowdenzug, mit dem die Verriegelungsvorrichtung entriegelbar ist, sodass das Kniegelenk, beispielsweise für den Übergang in die sitzende Position des Patienten, gebeugt werden kann. Auch wenn der Bowdenzug in eine griffgünstige Position geführt ist, ist seine Betätigung doch umständlich und erfordert beispielsweise bei einer Beinorthese den Griff ans Bein unter die Kleidung oder in die Kleidung hinein, was von vielen Patienten als störend empfunden wird.

Durch EP 0 141 640 A1 ist eine Kniegelenkprothese bekannt, bei der eine Entriegelung des künstlichen Gelenks elektromechanisch mittels einer in der Prothese angeordneten Steuereinheit erfolgt, die mit einem mit der Steuereinheit verbundenen Schalter betätigbar ist. Auch hier ist die Betätigung der Entriegelung umständlich und weist die oben beschriebenen Nachteile auf.

Durch EP 0 380 060 A2 ist es bekannt, die Bewegung einer Orthese mit drahtlos übertragenen Befehlen zu steuern, beispielsweise mit Sprachbefehlen "Aufwärts" und "Abwärts". Die Sprachbefehle werden in einer Steuereinrichtung in digitale Steuersignale umgesetzt und drahtlos auf ein an der Orthese befindliches Steuermodul übertragen. Das Steuermodul steuert einen Aktuator entsprechend den übertragenen Steuersignalen.

Der vorliegenden Erfindung liegt daher die Problemstellung zugrunde, ein orthopädietechnisches Hilfsmittel der eingangs erwähnten Art so auszubilden, dass eine ordnungsgemäße Entriegelung in einfacherer Weise erfolgen kann

Zur Lösung dieser Aufgabe ist erfindungsgemäß ein orthopädietechnisches Hilfsmittel der eingangs erwähnten Art dadurch gekennzeichnet, dass ein Betätigungssignal von einer in eine Gehhilfe integrierten Betätigungseinheit aus drahtlos auf das Steuermodul übertragbar ist.

Das erfindungsgemäße orthopädietechnische Hilfsmittel sieht somit eine elektromechanische Betätigung der Verriegelungsvorrichtung zum Entriegeln vor, die von einem Steuermodul ausgelöst wird. Das Steuermodul ist mit einem Signalempfänger versehen, durch den das Steuermodul ein drahtlos übermitteltes Betätigungssignal empfangen und in ein Schaltsignal für die Betätigung der Verriegelungsvorrichtung umsetzen kann. Die Betätigungseinheit ist in eine Gehhilfe integriert.

Dabei kann bevorzugt die Betätigungseinheit in einem Griff der Gehhilfe untergebracht sein. Günstig ist dabei die Anordnung eines Betätigungsknopfes an einer freien Stirnseite des Griffes der Gehhilfe, sodass der Betätigungsknopf vorzugsweise mit dem Daumen der den Griff haltenden Hand betätigt werden kann, ohne die Festigkeit des Griffes an der Gehhilfe verringern zu müssen.

Die Betätigungseinheit kann auch durch einen Handsender gebildet sein, der separat getragen und betätigt werden kann. Vorzugsweise ist der Handsender so ausgebildet, dass er in eine Gehhilfe an den vorgenannten Stellen eingesetzt und bei Benutzung der Gehhilfe betätigt werden kann, wobei der Handsender vorzugsweise im Griff der Gehhilfe untergebracht ist und vorzugsweise mit dem Daumen der den Griff haltenden Hand betätigt werden kann.

In einer weiteren Ausführung der Erfindung kann ein Quittungs- oder Warnsignal von dem Steuermodul auf die Betätigungseinheit übertragbar sein. Das Quittungssignal kann in einer bevorzugten Ausführung der Erfindung anzeigen, dass nach einer Entriegelung des orthopädietechnischen Hilfsmittels dieses wieder in die ordnungsgemäß verriegelte Position gelangt ist. Dies ist insbesondere für Beinorthesen von Bedeutung, deren Funktion es ist, in einer gestreckten Stellung des Kniegelenks zum Gehen verwendet zu werden. Mit dem übertragenen Quittungs- oder Warnsignal können Signaleinrichtungen der Betätigungseinheit angesteuert werden, beispielsweise optische und/oder akustische Signalanzeigeeinrichtungen und/oder ein Vibrator.

Für die in den Griff der Gehhilfe integrierte Betätigungseinheit ist dabei die Anbringung des Vibrators in dem Griff der Gehhilfe besonders zweckmäßig.

Die Erfindung soll im Folgenden anhand eines in der Zeichnung dargestellten Ausführungsbeispiels näher erläutert werden. Es zeigen:
- Figur 1: eine Seitenansicht einer Beinorthese gemäß einer Ausführungsform der Erfindung
- Figur 2: eine zu der Beinorthese gemäß Figur 1 gehörende Gehhilfe in Form einer Stütze
- Figur 3: eine Detaildarstellung eines Gelenks der Orthese gemäß Figur 1 im gesperrten Zustand in einer Seitenansicht
- Figur 4: das Gelenk gemäß Figur 3 in einer Ansicht von hinten
- Figur 5: die Ansicht gemäß Figur 3 für das Gelenk im entriegelten Zustand
- Figur 6: die Ansicht gemäß Figur 4 für das Gelenk im entriegelten Zustand
- Figur 7: eine Seitenansicht des Gelenks gemäß Figur 5 im gebeugten Zustand
- Figur 8: eine Seitenansicht eines Steuermoduls mit einer Entriegelungstaste
- Figur 9: eine Draufsicht auf das Steuermodul gemäß Figur 8
- Figur 10: ein Blockschaltbild für die elektrischen Teile der Beinorthese gemäß Figur 1, die mit einem Steuermodul gemäß den Figuren 8 und 9 ausgerüstet ist
- Figur 11: ein Steuermodul für die Beinorthese gemäß Figur 1, die drahtlos ansteuerbar ist
- Figur 12: eine Draufsicht auf das Steuermodul gemäß Figur 11

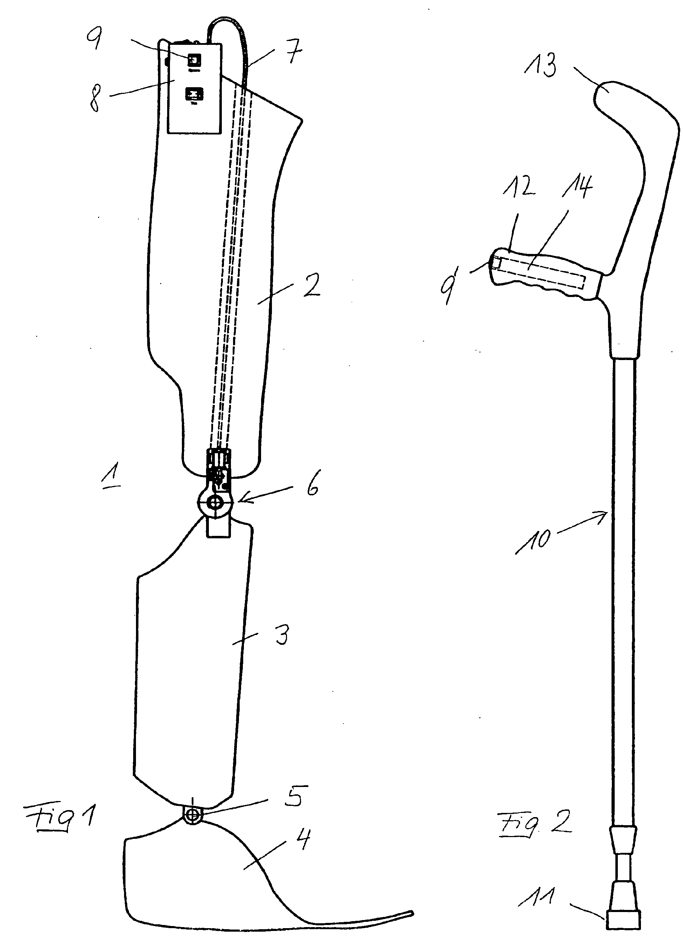

Die in Figur 1 dargestellte Beinorthese 1 weist eine Oberschenkelschale 2, eine Unterschenkelschale 3 und einen Fußaufnahmeschale 4 auf. Unterschenkelschale 3 und Fußaufnahmeschale 4 sind durch ein Drehgelenk 5 miteinander verbunden. Zwischen der Oberschenkelschale 2 und der Unterschenkelschale 3 ist ein verriegelbares Gelenk 6 angeordnet, das über ein Verbindungskabel 7 mit einem an der Oberseite der Oberschenkelschale 2 einhängbares Steuermodul 8 verbunden ist.

In Figur 1 nicht dargestellt sind in das Gelenk 6 einsteckbare flache Versteifungsstäbe, die mit der Obeschenkelschale 2 bzw. der Unterschenkelschale 3 verbindbar sind.

Das Gelenk 6 ist in der in Figur 1 dargestellten gestreckten Stellung verriegelbar und mittels einer Entriegelungstaste 9 des Steuermoduls 8 entriegelbar.

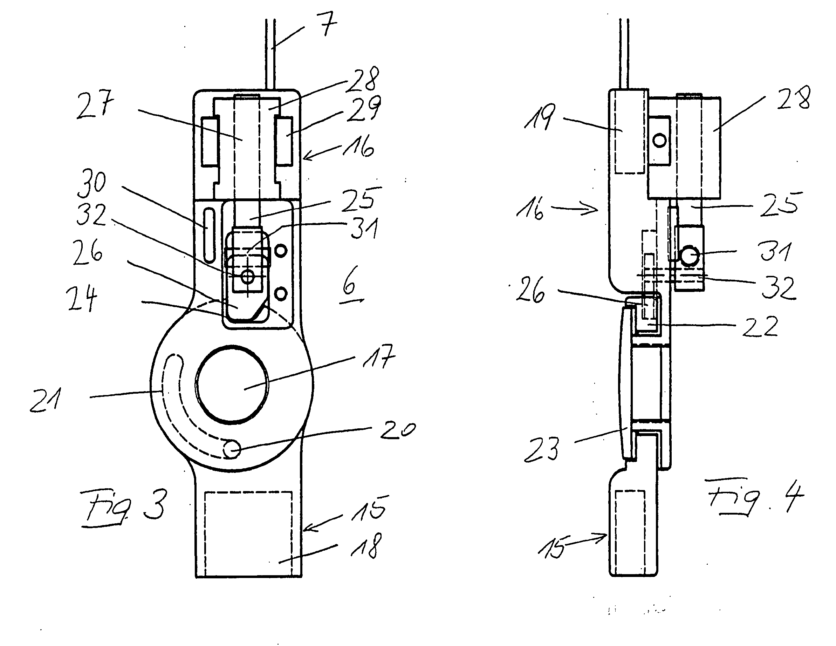

Figur 2 zeigt eine Gehhilfe 10 in Form einer stangenförmigen Stütze, die am unteren Ende ein zum Aufsetzen auf dem Boden vorgesehenes Gummiteil 11 und am oberen Ende einen Griff 12 und eine Unterarmstütze 13 aufweist. In den Griff 12 ist ein Auslöseschalter 9' integriert, der von der stirnseitigen Endfläche des Griffs 12 vorzugsweise mit dem Daumen betätigbar ist und der auf einen Sender 14 einwirkt, der daraufhin ein Betätigungssignal für das Steuermodul 8 aussenden kann. Das Steuermodul 8 ist in diesem Fall für einen Funkempfang eingerichtet.

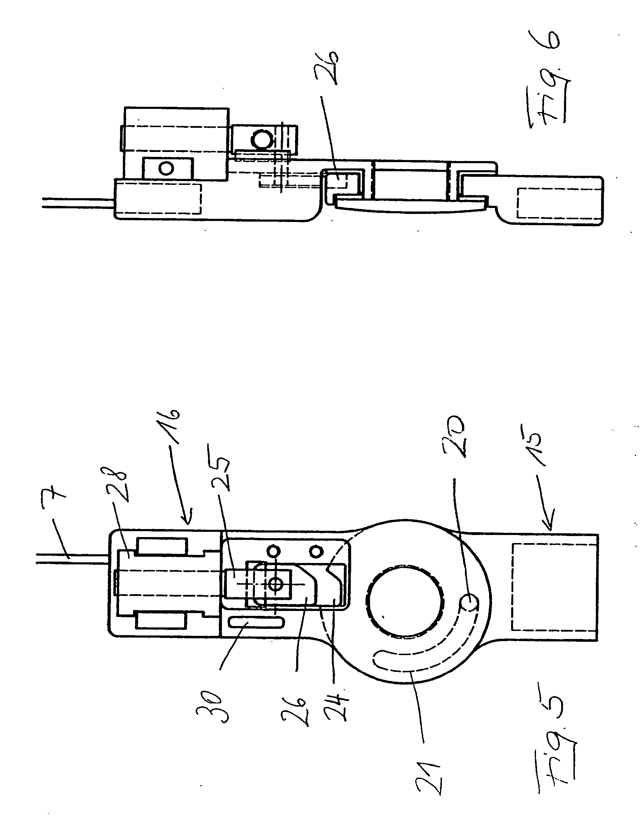

Der Aufbau des Gelenks 6 ist in den Figuren 3 bis 7 näher dargestellt. Das Gelenk 6 besteht aus zwei Gelenkteilen 15, 16 die über das Drehgelenk 17 drehbar miteinander verbunden sind.

Das Teil 15 ist als Gelenkunterteil mit einer nach unten offenen Aufnahmekammer 18 für einen flachen Versteifungsstab versehen, der mit der Unterschenkelschale 3 verbunden wird. In entsprechender Weise weist das Teil 16 eine nach oben offene Aufnahmekammer 19 zur Aufnahme eines Versteifungsstabs für die Oberschenkelschale 2 auf.

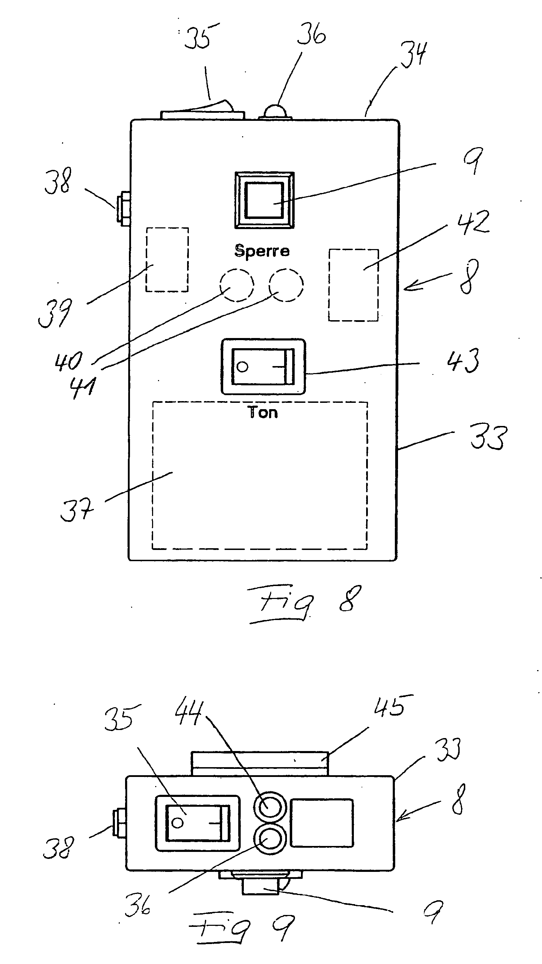

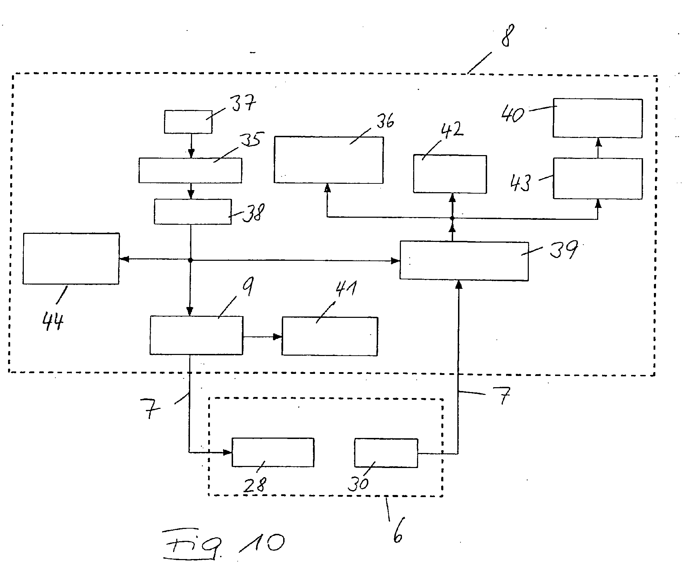

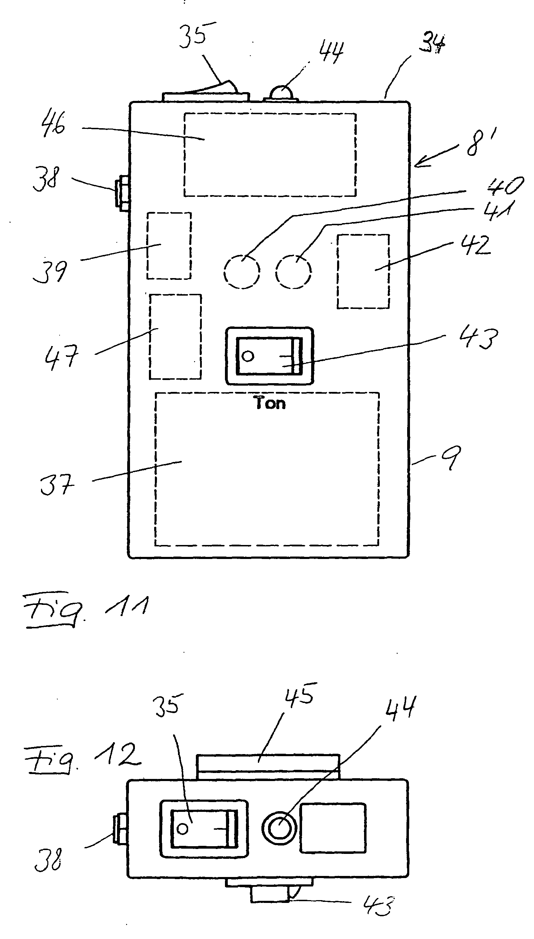

Das Gelenkunterteil 15 ist mit einem Führungsstift 20 versehen, der in einer etwa einen Viertelkreis ausbildenden Führungsnut 21 bewegbar ist und so Anschläge für die gestreckte Stellung gemäß Figur 3 und eine gebeugte Stellung gemäß Figur 7 des Gelenks 6 ausbildet.

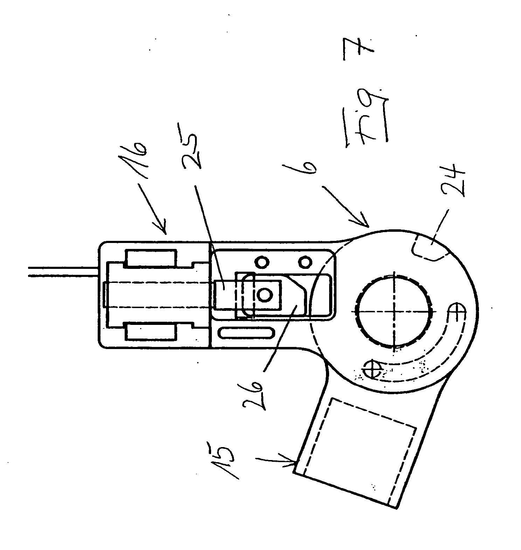

Gelenkunterteil 15 und Gelenkoberteil 16 bilden im Bereich des Drehgelenks 17 beide kreisförmige, augenartige Endabschnitte 22, 23 aus, die ineinander zur Bildung des Drehgelenks 17 montiert sind. Der Endabschnitt 22 des Gelenkunterteils 15 ist mit einer radialen Ausnehmung 24 versehen, in die ein Verriegelungsstift 25 mit einem unteren, komplementär zur Ausnehmung 24 gefomten Ende 26 eingreift, um Gelenkunterteil 15 und Gelenkoberteil 16 in der in Figuren 3 und 4 dargestellten gestreckten Stellung miteinander zu verriegeln. Der Verriegelungsstift 25 geht an seinem oberen Ende in einen zylindrischen Kern 27 über, der im Innenraum einer elektrischen Spule 28 axial bewegbar ist. Die elektrische Spule 28 ist in einer Halterung 29 im Gelenkoberteil fixiert. Die Position des Verriegelungsstifts 25 ist mittels eines im Gelenkoberteil 16 neben dem Verriegelungsstift 25 angeordnete und sich parallel zu ihm erstreckenden Sensor 30 detektierbar. Mit dem Sensor 30 wirkt ein mit dem Verriegelungsstift 25 verbundender Permanentmagnet 31 zusammen, der sich quer zum Verriegelungsstift 27 erstreckt und dessen Magnetfeld durch den Sensor 30, der ein Hall-Sensor sein kann, detektierbar ist. In der in Figur 3 dargestellten verriegelten Stellung detektiert der Sensor 30 kein Magnetfeld des Permanentmagneten 31. Bewegt sich der Verriegelungsstift 25 nach oben, weil er aufgrund eines Stromflusses durch die Spule 28 in deren Innenraum gezogen wird, gelangt das Feld des Permanentmagneten 31 in den Bereich des Sensors 30, der somit den entriegelten Zustand detektiert. Sowohl der Strom für die Spule 28 als auch das Ausgangssignal des Sensors 30 werden über das Verbindungskabel 7 von bzw. zu dem Steuermodul 8 übertragen.

Figur 4 verdeutlicht, dass das Endstück 26 des Verriegelungsstifts 25 axial seitlich versetzt verfahrbar ist und mit dem Verriegelungsstift 25 über einen Verbindungssift 32 verbunden ist.

Die Figuren 5 und 6 zeigen das Gelenk 6 im entriegelten Zustand. Über das Verbindungskabel 7 wird die Spule 28 von Strom durchflossen und wirkt als Elektromagnet für den Verriegelungsstift 25, der in das Innere der Spule 28 - in der zeichnerischen Darstellung also nach oben - gezogen wird und das mit ihm verbundene Ende 26 aus der zugehörigen Ausnehmung 24 herauszieht, sodass das Gelenkunterteil 15 nunmehr gegenüber dem Gelenkoberteil 16 drehbar ist, und zwar im Rahmen der durch den Führungsstift 20 und die Führungsnut 21 vorgegebenen Führung 20, 21.

Figur 7 zeigt die gebeugte Endstellung des Gelenks 6, wie sie eingenommen wird, wenn sich der Benutzer setzt. Das Ende 26 des Verriegelungsstifts 25 gleitet dabei auf der zylinderischen Umfangsfläche des Endabschnitts 22 des Gelenkunterteils 15. Geht der Benutzer aus dem gebeugten Zustand gemäß Figur 7 in den gestreckten Zustand gemäß Figuren 3 bis 6 über, gleitet das untere Ende 26 des Verriegelungsstifts 15 unter dem Einfluss der Schwerkraft auf der Umfangsfläche des Endabschnitts 22, bis das untere Ende 26 in der vollständig gestreckten Stellung in die Ausnehmung 24 hineinfällt und die Verriegelung gemäß den Figuren 3 und 4 wirkt.

Das in den Figuren 8 und 9 dargestellte Steuermodul 8 weist die Taste 9 zum Entriegeln des Gelenks 6 an einer bequem erreichbaren Stelle auf. Das Steuermodul ist mit einem rechteckigen, flachen Gehäuse 33 versehen an dessen schmaler Oberseite 34 ein Hauptschalter 35 mit einer Warnleuchte 36 angeordnet ist. Das Gehäuse 33 beinhaltet an der Unterseite eine Batterie 37, die über eine an einer schmalen Seitenwand angebrachte Ladebuchse 38 wiederaufladbar ist. Das Steuermodul 8 beinhaltet ferner eine Kurzzeitsteuerung 39 und zwei Tongeneratoren 40, 41 sowie einen Vibrator 42. Über einen Schalter 43 können die Tongeneratoren 40, 41 abgeschaltet werden, um ein in bestimmten Situationen unerwünschtes akustisches Signal zu unterdrücken.

Figur 9 lässt erkennen, dass an der Oberseite 34 des Gehäuses 33 eine weitere Kontrollleuchte 44 vorgesehen ist, die den Ladezustand der Batterie 37 anzeigt. Ferner ist das Gehäuse 33 mit einem Klemmbügel 45 versehen, mit dem es an der Oberkante der Oberschenkelschale 2 klemmend befestigbar ist.

Das in Figur 10 dargestellte Blockschaltbild zeigt die funktionale Verschaltung in dem Steuermodul 8 und die auf das Gelenk 6 über das Verbindungskabel 7 übertragenen Signale.

Die Batterie 37 ist über den Hauptschalter 35 mit der Ladebuchse 38 und der Taste 9 zum Entriegeln des Gelenks 6 verbunden. Wird die Taste 9 betätigt, wird damit der Tongenerator 41 angesteuert, der ein Warnton für die Entriegelung abgibt. Bei eingeschalteten Hauptschalter 35 wird der Ladezustand der Batterie 37 durch die Kontrollleuchte 44 angezeigt, indem die Kontrollleuchte 44 beispielsweise nicht aufleuchtet, wenn der Ladezustand der Batterie 37 ausreichend ist. Durch die Taste 9 wird ein Strom über das Verbindungskabel 7 in die Spule 28 im Gelenk 6 geleitet, wodurch die Entsperrung vorgenommen wird.

Detektiert der Sensor 30 des Gelenks 6, dass der Verriegelungsstift 25 wieder in die verriegelte Stellung gefallen ist, wird dieses Ausgangssignal des Sensors 30 über das Verbindungskabel 7 in das Steuermodul 8 übertragen und löst dort über die Kurzzeitsteuerung 39 Quittungssignale aus, nämlich durch ein über die Kurzzeitsteuerung 39 gesteuertes Aufleuchten der Kontrollleuchte 36, Betätigen des Vibrators 42 und Betätigen des Tongenerators 40, sofern dieser nicht über den Schalter 43 abgeschaltet worden ist.

Die Figuren 11 und 12 zeigen das Steuermodul 8' in einer für einen Funkempfang von dem Sender 14 der Gehhilfe 10 ausgelöstes Betätigungssignal und ist daher zusätzlich mit einem Funkempfänger 46 und einem Schaltrelais 47 anstelle des Schalters 9 versehen. Selbstverständlich ist es auch möglich, zusätzlich zu dem Funkempfänger 46 und dem Schaltrelais 47 auch die Entriegelungsmöglichkeit über die Taste 9 in dem Steuermodul 8, 8' vorzusehen.

In der in den Figuren 11 und 12 dargestellten Ausführungsform ist wegen des Wegfalls des Schalters 9 auch die entsprechende Kontrollleuchte 36 entfallen, sodass sich an der Oberseite 34 des Gehäuses 9 nur noch die Kontrollleuchte 44 für den Ladezustand der Batterie 37 befindet.

Im Übrigen ist das Steuermodul 8' identisch zum Steuermodul 8 aufgebaut.

## Patentansprüche

1. Orthopädietechnisches Hilfsmittel mit zwei relativ zueinander bewegbaren Teilen (15, 16) und einer Verriegelungsvorrichtung zum Verriegeln der beiden Teile (15, 16) an einer vorbestimmten relativen Position und zum Entriegeln der Teile (15, 16) zur Freigabe der Bewegung der Teile (15, 16) zueinander, wobei die Verriegelungsvorrichtung von einem Steuermodul (8, 8') aus elektromechanisch betätigbar ist, **dadurch gekennzeichnet, dass** ein Betätigungssignal von einer in eine Gehhilfe (10) integrierten Betätigungseinheit (9', 14) aus drahtlos auf das Steuermodul (8, 8') übertragbar ist.

2. Orthopädietechnisches Hilfsmittel nach Anspruch 1, **dadurch gekennzeichnet, dass** die Betätigungseinheit (9', 14) in einem Griff (12) der Gehhilfe (10) untergebracht ist.

3. Orthopädietechnisches Hilfsmittel nach Anspruch 2, **dadurch gekennzeichnet, dass** ein Betätigungsknopf (9') an einer freien Stirnseite des Griffs (12) angeordnet ist.

4. Orthopädietechnisches Hilfsmittel nach Anspruch 1, **dadurch gekennzeichnet, dass** die Betätigungseinheit durch einen Handsender gebildet ist, der in die Gehhilfe (10) einsetzbar und dort betätigbar ist.

5. Orthopädietechnisches Hilfsmittel nach einem der Ansprüche 1 bis 4 **dadurch gekennzeichnet, dass** ein Quittungs- oder Warnsignal von dem Steuermodul (8, 8') auf die Betätigungseinheit (9', 14) übertragbar ist.

6. Orthopädietechnisches Hilfsmittel nach Anspruch 5 **dadurch gekennzeichnet, dass** die Betätigungseinheit (9', 14) eine durch das Quittungs- oder Warnsignal steuerbare optische und/oder akustische Signalanzeigeeinrichtung und/oder einen Vibrator aufweist.

7. Orthopädietechnisches Hilfsmittel nach Anspruch 6, **dadurch gekennzeichnet, dass** der Vibrator in einem Griff (12) der Gehhilfe (10) angeordnet ist.

## Claims

1. An orthopedic aid with two parts (15, 16) which are movable relative to one another and with a locking device for locking the two parts (15, 16) in a predetermined relative position and for unlocking the parts (15, 16) in order to permit movement of the parts (15, 16) with respect to one another, wherein the locking device can be actuated electromechanically from a control module (8, 8'), **characterized in that** an actuating signal can be sent by wireless transmission from an actuating unit (9', 14) being integrated into a walking aid (10) to the control module (8, 8').

2. The orthopedic aid as claimed in claim 1, **characterized in that** the actuating unit (9', 14) is accommodated in a handgrip (12) of the walking aid (10).

3. The orthopedic aid as claimed in claim 2, **characterized in that** an actuating button (9') is arranged on a free end face of the handgrip (12).

4. The orthopedic aid as claimed in claim 1, **characterized in that** the actuating unit is formed by a manual transmitter which can be fitted into the walking aid (10) and can be actuated there.

5. The orthopedic aid as claimed in one of claims 1 to 4, **characterized in that** an acknowledgement signal or warning signal can be transmitted from the control module (8, 8') to the actuating unit (9', 14).

6. The orthopedic aid as claimed in claim 5, **characterized in that** the actuating unit (9', 14) has a visual and/or acoustic signal indication arrangement and/or a vibrator that can be controlled by the acknowledgement signal or warning signal.

7. The orthopedic aid as claimed in claim 6, **characterized in that** the vibrator is arranged in a handgrip (12) of the walking aid (10).

## Revendications

1. Dispositif d'aide technique orthopédique comprenant deux parties (15, 16) mobiles l'une par rapport à l'autre et un dispositif de verrouillage pour verrouiller les deux parties (15, 16) dans une position relative prédéterminée et pour déverrouiller les parties (15, 16) pour libérer le mouvement des parties (15, 16) l'une par rapport à l'autre, dans lequel le dispositif de verrouillage peut être actionné électromagnétiquement à partir d'un module de commande (8, 8') **caractérisé en ce qu'**un signal d'actionnement peut être transmis vers le module de commande (8, 8') par une liaison sans fil à partir d'une unité d'actionnement (9', 14) intégrée dans une béquille (10).

2. Dispositif d'aide technique orthopédique selon la revendication 1, **caractérisé en ce que** l'unité d'actionnement (9', 14) est incorporé dans une poignée (12) de la béquille (10).

3. Dispositif d'aide technique orthopédique selon la revendication 2, **caractérisé en ce qu'**un bouton d'actionnement (9') est disposé sur un côté frontal libre de la poignée (12).

4. Dispositif d'aide technique orthopédique selon la revendication 1, **caractérisé en ce que** l'unité d'actionnement est constituée par un émetteur à commande manuelle pouvant être introduit dans la béquille (19) et être actionné dans cette position.

5. Dispositif d'aide technique orthopédique selon l'une des revendications 1 à 4, **caractérisé en ce qu'**un signal de reçu ou d'avertissement peut être transmis du module de commande (8, 8') vers l'unité d'actionnement (9', 14).

6. Dispositif d'aide technique orthopédique selon la revendication 5, **caractérisé en ce que** l'unité d'actionnement (9', 14) comporte un dispositif d'affichage optique et/ou acoustique et/ou un vibreur pouvant être commandé par le signal de reçu ou le signal d'avertissement.

7. Dispositif d'aide technique orthopédique selon la revendication 6, **caractérisé en ce que** le vibreur est disposé dans une poignée (12) de la béquille (10).
